# EUROPEAN PATENT APPLICATION

(11) **EP 4 621 072 A1**
(43) Date of publication of application: **24.09.2025**
(21) Application number: 23943152.1
(22) Date of filing: 27.09.2023
(51) Int. Cl.: C12Q 1/6886, C12Q 1/6816

(54) **METHOD FOR DETECTING METHYLATION STATUS OF DNA SAMPLE**

(30) Priority: 29.06.2023 CN 202310782685
(71) Applicant: Nanodigmbio (Nanjing) Biotechnology Co., Ltd, Nanjing, Jiangsu 210000 (CN)
(72) Inventor: YU, Liping, Nanjing, Jiangsu 210000 (CN); WANG, Biao, Nanjing, Jiangsu 210000 (CN); WU, Qiang, Nanjing, Jiangsu 210000 (CN)
(74) Representative: Botti & Ferrari S.p.A.
(86) International application number: PCT/CN2023/114353
(87) International publication number: WO 2025/000668

(57) **Abstract**

The present application provides a method for detecting the methylation status of a DNA sample, and specifically provides a method for detecting the methylation status of a DNA molecule in a sample, comprising: treating a sample with a methylation-sensitive restriction endonuclease or a methylation-dependent restriction endonuclease, comparing said sample with a sample not treated with a restriction endonuclease, and reflecting the methylation status of the DNA molecule by the degree of enzyme cutting. The present application also provides a kit for detecting the methylation status of a DNA molecule in a sample. The method for detecting the methylation status of a DNA molecule in a sample provided herein is simple in process and accurate in results, and can be used for early screening of multiple cancer types using ctDNA samples.

## Description

The present application claims priority to Chinese Patent Application No. 2023107826859 filed with the China National Intellectual Property Administration on June 29, 2023, which is hereby incorporated herein by reference in its entirety.

### TECHNICAL FIELD

The present disclosure relates to a method for detecting methylation, and in particular to a method for detecting methylation using a methylation-sensitive restriction endonuclease. The present disclosure also relates to a kit for detecting methylation.

### BACKGROUND

DNA methylation is one of the mechanisms by which cells regulate gene expression without altering gene sequences, thereby regulating protein expression. DNA methylation is associated with many physiological and pathological factors and carries a great deal of biological information. In cancer cells, the methylation status of DNA is altered, where hypomethylation of the proto-oncogene promoter region (activating carcinogenic factors) or hypermethylation of the anti-oncogene promoter region (downregulating the expression level of anti-oncogene) occurs. It has been reported that the methylation status of circulating tumor DNA (ctDNA) in plasma is consistent with that in cancer tissues, and that the methylation status of ctDNA can be traced to tissues. Therefore, it is advantageous to determine ctDNA methylation in cancer early screening projects.

There are various methods for methylation sequencing. Among them, first-generation and second-generation sequencing techniques can only distinguish four different bases A, T, C, and G, but cannot distinguish between methylated and unmethylated C bases. Conventional treatment protocols involves converting an unmethylated C base to a U base by bisulfite (BS) conversion, converting the U base to T by polymerase chain reaction (PCR) amplification, and comparing the amplified sequence with an unconverted sequence to determine whether methylation modification occurs at this site. Therefore, bisulfite conversion once became the gold standard for methylation sequencing. However, high salt and strong alkali conversion conditions used in BS conversion cause significant sample damage. For samples after conversion, the loss rate is about 70%. For samples such as ctDNA, which are difficult to acquire, a lot of methylation information will be lost. NEB (New England Biolabs) has developed an enzyme conversion kit according to the mechanism of action of methylation modification enzymes in organisms. By means of this conversion method, sequence information consistent with the BS conversion status is finally acquired. Using the principle of enzymatic conversion, the conversion-induced damage is relatively low, but enzymatic reactions are highly dependent on enzymatic activity, leading to incomplete conversion in complex regions. Meanwhile, multi-step conversion makes the operation rather cumbersome, and repeated purification steps will also lead to a certain degree of sample loss. Reduced Representation Bisulfite Sequencing (RRBS) is considered a cost-effective DNA methylation detection protocol, in which MspI endonuclease is used to cleave methylation-modified CCGG cleavage sites. The depth of coverage and ratio of RRBS correlate with the cleavage efficiency of MspI, making RRBS suitable for methylation detection across the whole genome of mammals. However, it is less suitable for methylation detection in samples related to early screening of cell free DNA (cfDNA).

At present, most of the methylation-based early-screening products that have been approved for marketing are mainly PCR products, which cover a limited number of sites, and mainly focus on the analysis of the methylation status of a single gene. With the development of the cancer early screening market, single-cancer early screening will transition to multi-cancer early screening. Higher demands are placed on cancer early screening technology. The methylation-targeted capture sequencing method is used to detect the methylation status of different sites by enriching the fragments of the target region. Depending on the targeted sequencing method, it is divided into liquid-phase hybridization capture sequencing technology and multiplex PCR amplification technology. The conventional liquid-phase hybridization capture technology faces challenges with the capture of a small methylated panel and is cumbersome in operation process. Multiplex PCR amplification technology necessitates rigorous primer design. A relatively large GC bias is present in the library after conversion, which further increases the difficulty of primer design. The development of methylation detection technology with simple operation process and high convenience, especially suitable for cfDNA samples, will facilitate the implementation of methylation-based early screening projects.

### SUMMARY

In one aspect, provided herein is a method for detecting a methylation status of a DNA molecule in a sample, including:
1) dividing the sample into two parts: a first sample and a second sample, where the first sample is treated with one or more methylation-sensitive restriction endonucleases(MSREs), such that an enzymatic cleavage reaction is performed in the absence of methylation of the DNA molecule at recognition sites of the MSREs, so as to obtain a cleaved sample; or the sample is treated with one or more methylation-dependent restriction endonucleases (MDREs), such that an enzymatic cleavage reaction is performed in the presence of methylation of the DNA molecule at recognition sites of the methylation-dependent restriction endonucleases, so as to obtain a cleaved sample; and the second sample is not treated with the restriction endonucleases;
2) contacting one or more capture probes with the cleaved sample, where the capture probes target a target sequence including the recognition sites in the DNA molecule, so as to isolate the non-cleaved DNA molecule including the recognition sites from the cleaved sample; contacting the capture probes with the second sample, so as to isolate the DNA molecule including the recognition sites from the second sample; and
3) making a comparison with an amount of the DNA molecule including the recognition sites in the second sample, and determining an overall methylation status of DNA molecules in the sample according to an amount of the non-cleaved DNA molecule including the recognition sites; or sequencing the non-cleaved DNA molecule including the recognition sites and the DNA molecule including the recognition sites in the second sample, and determining methylation statuses of different DNA molecules in the sample according to a ratio of sequencing depths.

In some embodiments, the capture probes include a target-specific sequence, a first probe-binding sequence at the 5' end of the target-specific sequence and a second probe-binding sequence at the 3' end of the target-specific sequence, the first probe-binding sequence being at least partially complementary to the second probe-binding sequence, such that when two or more capture probes bind to the target sequence in an adjacent manner, complementary binding between the adjacent capture probes is formed by the first probe-binding sequence and the second probe sequence.

In some embodiments, the first probe-binding sequence and the second probe-binding sequence are 8-30nt in length.

In some embodiments, the target-specific sequence is 20-80nt in length.

In some embodiments, before step 1), the method further includes constructing a sequencing library with the DNA molecule in the sample and, optionally, fragmenting the DNA molecule before constructing the sequencing library.

In some embodiments, the MSREs are selected from the group consisting of Hpa I, Hpa II, Hha I, Aci I, and any combination thereof.

In some embodiments, the methylation-dependent restriction endonucleases are selected from the group consisting of FspE I, LpnP I, MspJ I, and any combination thereof.

In some embodiments, the sample includes ctDNA and/or genomic DNA (gDNA), or the sample is a formalin-fixed paraffin-embeded (FFPE) sample.

In one aspect, provided herein is a kit for detecting the methylation status of a DNA molecule in a sample, including:
1) one or more MSREs that cleave the DNA molecule in the absence of methylation of the DNA molecule at the recognition sites of the MSREs; or one or more MDREs that cleave the DNA molecule in the presence of methylation of the DNA molecule at the recognition sites of the MDREs; and
2) one or more capture probes that include a target-specific sequence, a first probe-binding sequence at the 5' end of the target-specific sequence and a second probe-binding sequence at the 3' end of the target-specific sequence, the first probe-binding sequence being at least partially complementary to the second probe-binding sequence, such that when two or more capture probes bind to a target sequence including the recognition sites in the DNA molecule in an adjacent manner, complementary binding between the adjacent capture probes is formed by the first probe-binding sequence and the second probe sequence.

In some embodiments, the kit further includes an enzyme and an adapter molecule for constructing a sequencing library for the DNA molecule.

In some embodiments, the first probe-binding sequence and the second probe-binding sequence are 8-30nt in length.

In some embodiments, the target-specific sequence is 20-80nt in length.

In some embodiments, the MSREs are selected from the group consisting of Hpa I, Hpa II, Hha I, Aci I, and any combination thereof.

In some embodiments, the MDREs are selected from the group consisting of FspE I, LpnP I, MspJ I, and any combination thereof.

In some embodiments, the sample includes ctDNA.

The method for detecting a methylation status of a DNA molecule in a sample provided herein reflects the methylation level by restriction endonuclease cleavage levels. By integrating capture probes, the method features a simple process and accurate results, and thus can be used for early screening of multiple cancer types using ctDNA samples.

### BRIEF DESCRIPTION OF THE DRAWINGS

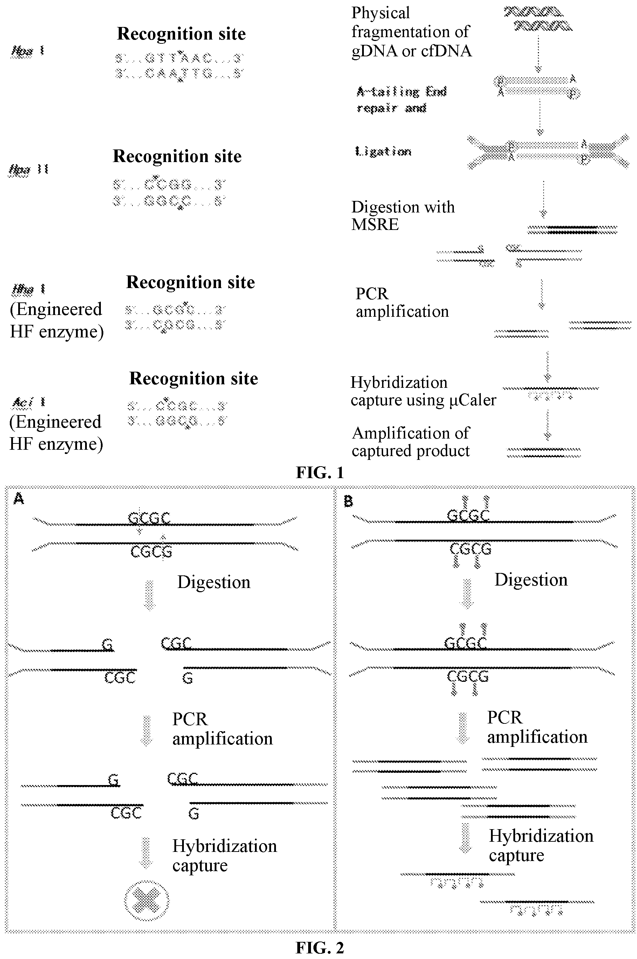

FIG. 1: Recognition sites for four MSREs and a construction process of targeted methylation capture library based on the MSREs. This process is suitable for the detection of methylation statuses in target regions of gDNA samples after physical fragmentation, plasma cfDNA or ctDNA samples.
FIG. 2: The results of library capture corresponding to the target regions with different methylation statuses after enzymatic cleavage with MSREs.

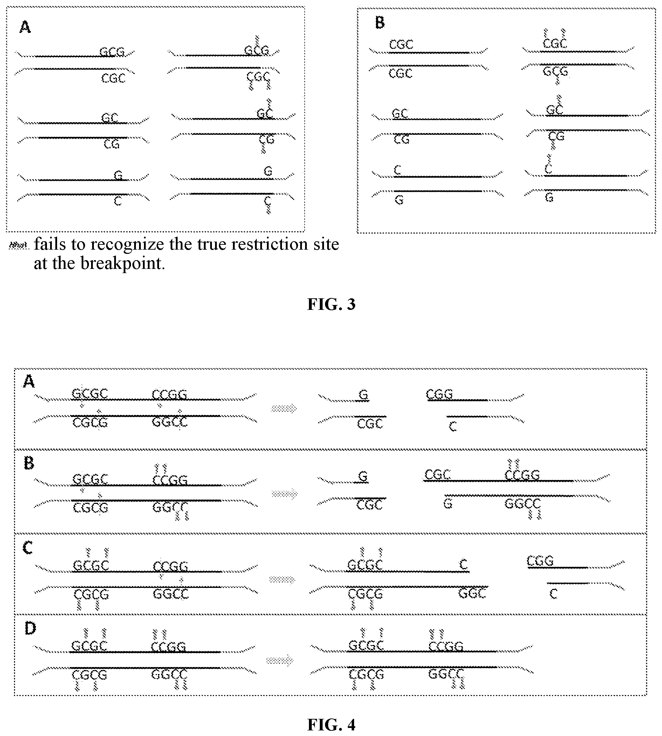

FIG. 3: The case where the constructed library does not contain an intact restriction endonuclease recognition site. The constructed library should contain an intact restriction endonuclease recognition site. When information on only 3 or less base positions in the cleavage site is retained in the library, the methylation status of this cleavage site cannot be determined. Hha I restriction site is taken as an example for explanation here. For this restriction site, this part of the data needs to be filtered out during the analysis. Panel A. The first 3 or 2 or 1 base of the restriction site is ligated to the adapter; Panel B. The last 3 or 2 or 1 base after the restriction site is ligated to the adapter.
FIG. 4: The case where the methylation status of a cleavage site cannot be determined due to the presence of two sensitive endonuclease recognition sites at one locus. (A) Both Hha I and Hpa II cleavage sites are unmethylated, in which case cleavage occurs; (B) Hha I cleavage site is unmethylated, while Hpa II cleavage site is methylated, in which case cleavage occurs; (C) Hha I cleavage site is methylated, while Hpa II cleavage site is unmethylated, in which case cleavage occurs; (D) Both Hha I and Hpa II cleavage sites are methylated, in which case cleavage does not occur.

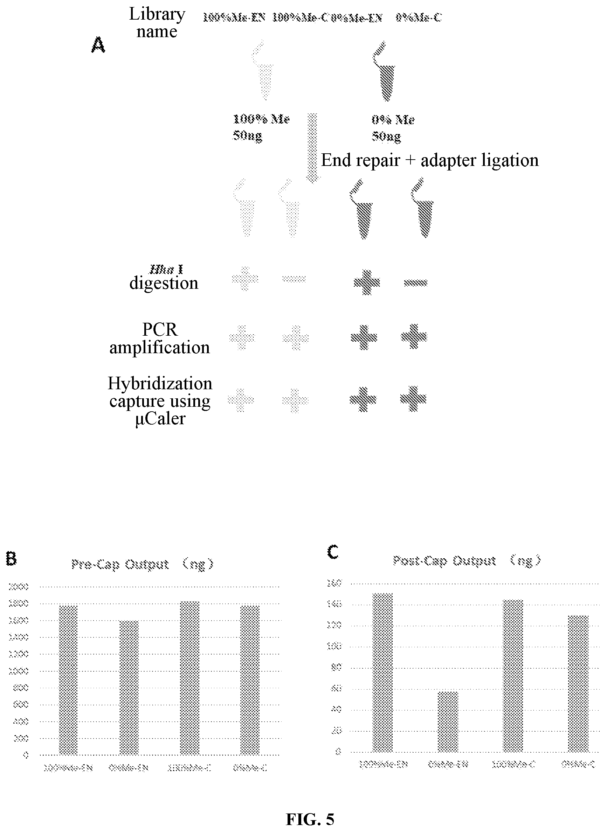

FIG. 5: Process of the construction of different libraries using two standards, 100% Methylated DNA and 0% Methylated DNA (ZYMO) (A) and library construction output of four libraries (B, pre-capture library yields; C, hybridization capture library yields).

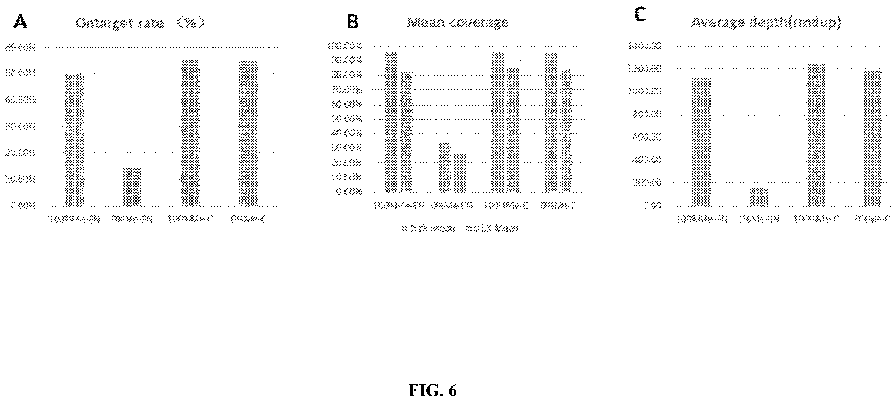

FIG. 6: On-target rate, coverage, and sequencing depth in libraries constructed from samples with different methylation statuses. The sample with a 0% methylation status has a reduced number of targets after cleavage, and the on-target rate, coverage and sequencing depth in the library are all lower than those in the other experimental groups. (A) [On-Target] Fraction of Target Reads in mapped reads; (B) 0.5× Mean Coverage; 0.2× Mean Coverage; (C) Average depth (rmdup).

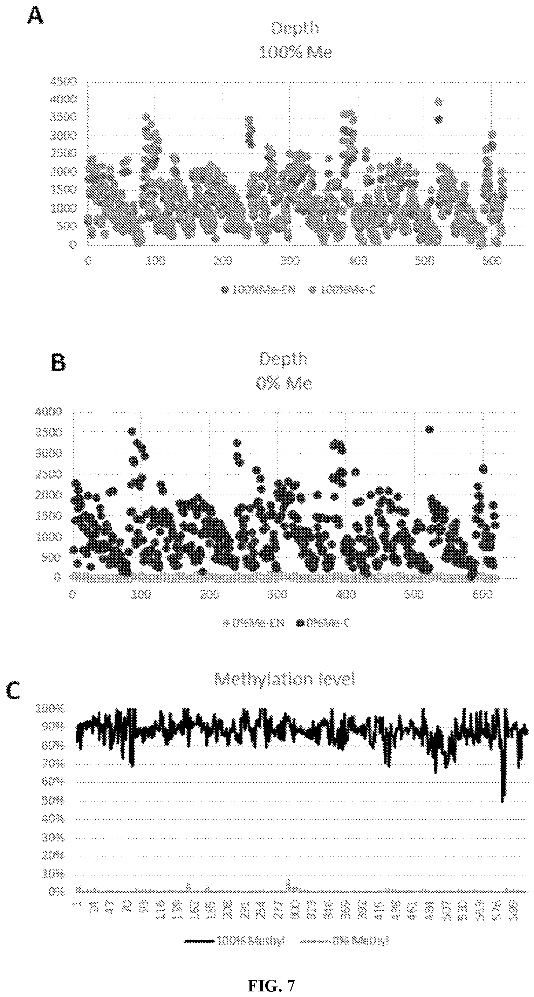

FIG. 7: Sequencing depth and corresponding methylation levels at CpG sites related to MSRE in target regions of different libraries. (A) Sequencing depths at CpG sites in cleavage and non-cleavage libraries of the samples with a 100% methylation level, in which 100% Me-EN represents cleavage library of samples with a100% methylation level, and 100% Me-C represents non-cleavage control library of samples with 100% methylation level; (B) Sequencing depths corresponding to CpG sites in cleavage and non-cleavage libraries of the samples with a 0% methylation level, in which 0% Me-EN represents cleavage library of samples with a 0% methylation level, and 0% Me-C represents non-cleavage control library of samples with a 0% methylation level; (C) Calculation method for methylation level: cleavage depth/control depth. The methylation level detected in the 0% Methyl sample is close to 0 and the methylation level detected in the 100% Methyl sample is close to 100%. The detection results are in line with expectations.

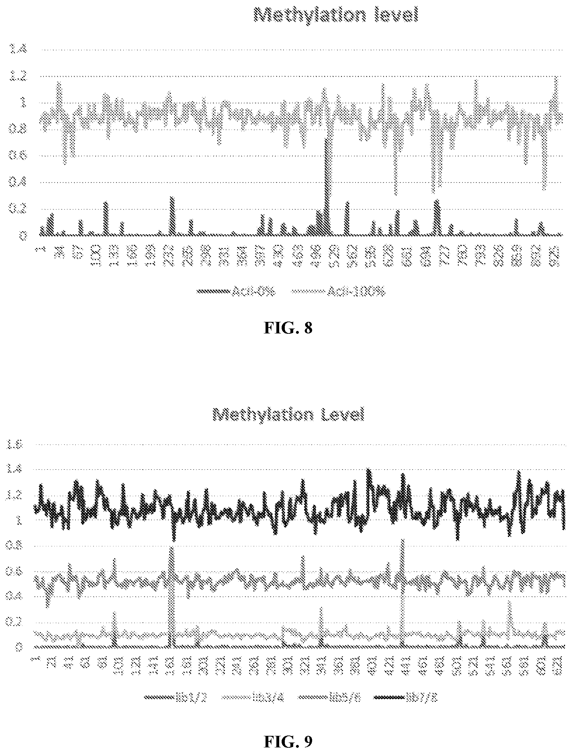

FIG. 8: Sequencing depth and corresponding methylation levels of CpG sites related to MSRE Aci I in target regions of different libraries. The methylation level detected in the 0% Methyl sample and the methylation level detected in the 100% Methyl sample are shown.
FIG. 9: Methylation levels of CpG sites related to MSRE in different samples. Calculation method for methylation level: cleavage depth/control depth. The methylation level detected in the 0% Methyl sample is close to 0 and the methylation level detected in the 10% Methyl sample is close to 10%. The methylation level detected in the 50% Methyl sample is close to 50%. The methylation level detected in the 100% Methyl sample is close to 100%. The detection results are all in line with the expectations.

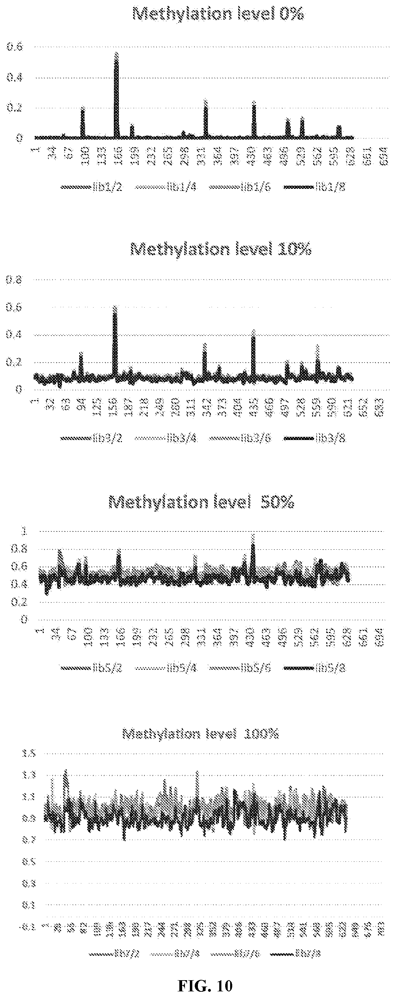

FIG. 10: Methylation levels of different samples are calculated for the cleaved samples, using non-cleaved samples with different methylation levels as controls.

### DETAILED DESCRIPTION OF THE EMBODIMENTS

Unless otherwise stated, all technical and scientific terms used herein have the meaning as commonly understood by those of ordinary skill in the art.

The term "or" refers to a single element of the listed selectable elements, unless otherwise clearly indicated in the context. The term "and/or" refers to any one, any two, any three, any more, or all of the listed selectable elements.

As used herein, the terms "comprise", "contain", "have" and similar expressions mean that elements not listed are not excluded. These terms also include cases consisting only of the listed elements.

As used herein, "DNA molecule" refers to deoxyribonucleic acid, which is a polymer of deoxyribonucleotides. It may be in a double-stranded form or in a single-stranded form. Alternatively, for a population of DNA molecules, it may include double-stranded DNA molecules or single-stranded DNA molecules. DNA molecules may be of any length, and for example, they may be genomic DNA, DNA fragments or cell-free DNA. Cell-free DNA refers to DNA that is free outside the cell or outside the cell nucleus and can be extracted from biological materials such as various body fluids and *in-vitro* cell culture media, or natural environment, including but not limited to: peripheral blood, plasma, serum, urine, feces, saliva, cerebrospinal fluid, lymphatic fluid, etc. Cell-free DNA can be obtained through extraction and purification. As used herein, "DNA fragments" refer to relatively short DNA molecules, for example, between 50 bp and 700 bp in length, for example, between 100 bp and 500 bp in length, especially between 100 bp and 350 bp in length. DNA fragments contained in biological samples are usually heterogeneous, i.e., of varying lengths. Correspondingly, the length described above can be referred to as the mean length of these DNA fragments. These DNA fragments may have different sequences, for example, those from different regions of the genome of the same organism, or even from different organisms. DNA fragments may be those with single-stranded nicks, or they may have blunt ends or non-blunt ends (with 3' overhangs or 5' overhangs).

As used herein, "DNA methylation" refers to the modification of bases in DNA molecules or DNA fragments by methylation, especially the modification of cytosine to 5-methylcytosine (5mC). DNA methylation in vertebrates generally occurs at the CpG site (i.e., the site in the DNA sequence where cytosine is immediately followed by guanine), and is catalyzed by DNA methyltransferase to convert the cytosine to 5-methylcytosine. Most of the CpG sites in the human genome have been methylated. However, in certain specific regions, such as CpG islands rich in cytosine (C) and guanine (G), CpG sites are usually unmethylated. CpG methylation can affect the transcriptional activity of related genes. For example, methylation can suppress oncogenes, while demethylation stimulates the expression of certain oncogenes. In these cases, cancer may occur. In addition, a small number of cytosine bases are modified to 5-hydroxymethylcytosine (5hmC), 5-formylation (5fC), and 5-carboxylation (ScaC), though the occurrence rate of these modifications is less than that of 5mC. Herein, references to methylation may also refer to modifications to ShmC, 5fC, and 5caC, unless otherwise stated in the context.

As used herein, "cytosine methylation status" or "DNA methylation information" refers to information on the methylation status of DNA molecules or DNA fragments, including but not limited to, methylation sites, methylation levels, methylation patterns (e.g., 5mC or ShmC), etc. "Methylation level", also known as "methylation degree", refers to the proportion (or frequency) of methylation modification at a specific methylation site in a sample. A variety of ways can be used to detect whether a site is methylated. Common methods include chemical or enzymatic conversion methods. During conversion, either methylated cytosine or unmethylated cytosine is converted into uracil (U) or a base that is basically equivalent to uracil in terms of base pairing, such as dihydrouracil (DHU). During subsequent amplification, the corresponding uracil is paired with adenine (A) as thymine (T), and as a result, the cytosine or methylated cytosine at the methylation site is reflected as thymine in the detection result (such as the sequencing result). By aligning with the reference sequence, whether the cytosine in a DNA molecule or DNA fragment is methylated can be determined. The reference sequence may be a sequence from the same sample but not subjected to the conversion described above, or a corresponding sequence in a healthy population. Moreover, some methods can be used to distinguish 5mC from ShmC. Currently, DNA methylation information has been widely used in the screening and diagnosis, especially in early screening and diagnosis, of cancer (e.g., lung cancer, breast cancer, liver cancer, colorectal cancer, etc.). In addition, the identification of methylation statuses can be used for non-diagnostic (or therapeutic) purposes, for example, for scientific research, such as analysis of factors affecting methylation and the effect of methylation on gene function, for the detection of methylation standards, or the like.

"Methylation-sensitive restriction endonuclease (MSRE)" refers to a restriction endonuclease that is sensitive to whether a methylated base is present at its recognition site. Such enzymes, when one methylated base is present at their recognition sites, are generally unable to cleave DNA molecules at their recognition sites. As used herein, MSRE especially refers to a restriction endonuclease that, when at least one 5mC is present at its recognition site, is unable to cleave a DNA molecule at the recognition site. MSREs known in the art include, but are not limited to, Hpa I, Hpa II, Hha I and Aci I.

In contrast to "methylation-sensitive restriction endonuclease", "methylation-dependent restriction endonuclease (MDRE)" refers to a restriction endonuclease that depends on the presence of a methylated base at its recognition site. Such enzymes, when no methylated bases are present at their recognition sites, are generally unable to cleave a DNA molecule at their recognition sites. As used herein, methylation-dependent restriction endonuclease especially refers to a restriction endonuclease that, only when at least one 5mC is present at its recognition site, is able to cleave a DNA molecule at the recognition site. MDREs known in the art include, but are not limited to, FspE I, LpnP I and MspJ I.

As used herein, "capture probe" refers to a single-stranded DNA fragment used to hybridize to a target DNA molecule. Herein, the capture probe used preferably targets a target sequence containing a restriction endonuclease recognition site in a DNA molecule. For a DNA molecule treated with a specific restriction endonuclease, if the methylation status of the recognition site of the restriction endonuclease enables the restriction endonuclease to cleave the DNA molecule, the capture probe can bind only partially or insufficiently to the target DNA molecule, such that the target DNA molecule cleaved by the restriction endonuclease cannot be isolated from the sample. In this case, the target DNA molecules that can be isolated from a sample treated with the restriction endonuclease using capture probes are mainly uncleaved DNA molecules. Therefore, the overall methylation status of the DNA molecule in the sample can be determined according to the amount of the DNA molecule not subjected to cleavage that can be isolated relative to the amount of the DNA molecule that can be isolated in the sample that is not treated with restriction endonucleases. For example, under treatment with a MSRE, the amount of cleaved DNA molecules is relatively high (the amount of uncleaved DNA molecules is relatively low), indicating that the DNA molecules in the sample have a relatively low methylation level at the restriction endonuclease recognition site. In a preferred embodiment, the capture probe is a µCaler probe (the design of the µCaler probe can be found in Chinese Patent Publication No. CN 116083423 A, which is hereby incorporated herein by reference in its entirety). Such a probe includes a target-specific sequence, a first probe-binding sequence at the 5' end of the target-specific sequence and a second probe-binding sequence at the 3' end of the target-specific sequence, the first probe-binding sequence being at least partially complementary to the second probe-binding sequence, such that when two or more capture probes bind to the target sequence in an adjacent manner, complementary binding between adjacent capture probes can be formed by the first probe-binding sequence and the second probe-binding sequence. The benefits of using the µCaler probe over conventional capture probes at least lie in the significantly enhanced binding affinity to the target, improved efficiency of hybridization capture in the target region, and greater overall coverage uniformity and stability. Capture probes can typically equipped with a separable tag, such as biotin, to separate target DNA molecules bound to the probes. The sequence information of different target DNA molecules isolated can be obtained through sequencing, and the methylation status at the recognition site of the restriction endonuclease can also be obtained.

A process of the method for detecting a methylation status of a DNA sample provided herein can be found in FIG. 1. This process is suitable for the construction of methylation libraries for both genomic DNA and cell-free DNA in plasma. This process is briefly described as follows: Genomic DNA is fragmented into DNA fragments of 200-250 bp by ultrasonication. The fragmented samples are used for library construction, and the Nadprep Universal DNA Library Construction Kit can be used as the library construction kit. The library construction process includes A-tailing end repair and adapter ligation. The ligation products are divided into two groups. One group is cleaved with a MSRE (whose action is blocked by methylation), where the restriction endonuclease is selected from the group consisting of Hpa I, Hpa II, Hha I, Aci I, and the like, and the other group is left uncleaved and serves as a control group. PCR amplification is performed separately for the two groups of products, with the addition of Index sequences. The amplified products can be subjected to hybridization capture using the µCaler hybrid Capture system. All the probes are designed for DNA strands using the µCaler probe design scheme, which cover methylation-sensitive restriction sites. The captured libraries are sent to Illumina or MGI platform sequencer for sequencing.

Target regions with different methylation statuses are cleaved with MSREs, and the library capture results are shown in FIG. 2. In this schematic diagram, Hha I restriction endonuclease is taken as an example. FIG. 2A shows that no methylation modification occurs in the C base at the GCGC restriction endonuclease recognition site in the target region, and Hha I recognizes and cleaves the adapter ligation product having this site, such that this target cannot be detected during both PCR amplification and targeted capture. FIG. 2B shows that methylation modification occurs in the C base at the GCGC restriction endonuclease recognition site in the target region, and Hha I cannot cleave the adapter ligation product having this site, such that this target can be detected during both PCR amplification and targeted capture. The detection principle using other MSREs is consistent with this.

The constructed library should contain an intact restriction endonuclease recognition site. The breakpoints of gDNA or cfDNA after unltrasonication are random. If the breakpoints are located within the cleavage site, adapters are ligated to the breakpoints. When information on only 3 or less bases in the cleavage site is retained in the library, the methylation status of this cleavage site cannot be determined. This part of data needs to be filtered out during the analysis. As shown in FIG. 3, Hha I restriction site is taken as an example for explanation. For this restriction site, this part of data needs to be filtered out during the analysis. FIG. 3A shows that the first 3, 2 or 1 base of the restriction site is ligated to the adapter; FIG. 3B. The last 3, 2 or 1 base of the restriction site is ligated to the adapter.

The case where the methylation status of a cleavage site cannot be determined due to the presence of two sensitive endonuclease recognition sites at one locus is as shown in FIG. 4. This process allows for the simultaneous cleavage by two or more restriction endonucleases. When a combination of multiple restriction endonucleases is used, consideration needs to be given to the phenomenon of whether two cleavage sites are contained simultaneously in the same library fragment; if two restriction endonuclease recognition sites are contained simultaneously in the same DNA fragment, the methylation statuses of all sites cannot be accurately analyzed. Hha I and Hpa II restriction sites are taken as examples for explanation. Panel A shows that both Hha I and Hpa II cleavage sites are unmethylated, in which case cleavage occurs; Panel B shows that Hha I cleavage site is unmethylated, while Hpa II cleavage site is methylated, in which case cleavage can occur; Panel C shows that Hha I cleavage site is methylated, while Hpa II cleavage site is unmethylated, in which case cleavage occurs; Panel D shows that both Hha I and Hpa II cleavage sites are methylated, in which case cleavage does not occur. Among the above four statuses, only the status in Panel D can be detected. Statuses in panels A, B and C cannot be distinguished from each other. Therefore, the probe used in the method of the present disclosure should cover as much as possible a single restriction site to ensure the accuracy of the detection results.

Analysis protocol for methylation level: Methylation levels of different samples are calculated for the cleaved samples, using non-cleaved samples with different methylation levels as controls. Calculation method for methylation level: For each site, normalization is performed using the reference gene, and the methylation level is calculated according to formula: normalized depth of each CpG site in cleavage sample/normalized depth of each CpG site in control sample.

Therefore, provided herein is a process for constructing a targeted methylation library based on an MSRE, where the MSRE includes Hpa I, Hpa II, Hha I, Aci I, etc. In some embodiments, the targeted capture system captures the original DNA sequences in the unconverted library. When the C base in a CpG site of a restriction endonuclease recognition site in a target region of the captured fragment is methylated, the information on the methylation modification in the target region is detected. In some embodiments, the capture probe is designed for the original DNA strand, and the targeted capture probe needs to cover the corresponding MSRE cleavage site. According to the process for constructing a targeted methylation library based on the MSRE provided herein, the sequencing depths at CpG sites can be detected. In the system, a reference gene is introduced for normalization, and a control is used for analysis of the methylation degree at CpG sites in the sample.

### General Experimental Procedures:

### Step one: Sample fragmentation

DNA sample fragmentation is performed according to laboratory conditions. Covaris^{™} series DNA ultrasonicator is recommended for the fragmentation of samples into products with average fragment lengths of 200-250 bp. gDNA or FFPE DNA needs to be fragmented before proceeding to step two. cfDNA sample proceeds directly to step two.

### Step two: End Repair & A-Tailing

1. End Repair & A-Tailing Buffer is taken out and thawed at room temperature, mixed well and placed on ice for later use.
2. End Repair & A-Tailing Enzyme is taken out and placed on ice for natural thawing, mixed well and centrifuged briefly for later use.
3. A reaction system is formulated in a 0.2 mL PCR tube placed on ice according to the following table:

| | |
|---|---|
| Fragmented gDNA or cfDNA | 20 µL |
| End Repair & A-Tailing Buffer | 3 µL |
| End Repair & A-Tailing Enzyme | 2 µL |
| Total | 25 µL |

| | |
|---|---|
| Note: If the volume of DNA is less than 20 µL, Nuclease-Free Water can be used to make up the volume to 20 µL. | |

4. The reaction system is mixed well and centrifuged briefly to make all the reaction solution settle at the bottom of the PCR tube.
5. The following (Cycling Program I) reaction programs are initiated on a thermal cycler and the PCR tube is placed in the thermal cycler when the temperature is stabilized to 20°C.

| | |
|---|---|
| 20°C | 30 min |
| 65°C | 30 min |
| 10°C | Hold |

| | |
|---|---|
| Note: There is no need for the heated lid when the program is running at 20°C, and the heated lid needs to be set to 70°C when the program is running at 65°C. | |

### Step three: Adapter ligation

1. Ligation Buffer is taken out and thawed at room temperature, mixed well and placed on ice for later use.
Note: The Ligation Buffer is very viscous and therefore pipetting and dispensing should be slow and steady to ensure volume accuracy.
2. DNA Ligase is taken out and naturally thawed on ice, mixed well and centrifuged briefly for later use.
3. The PCR tube from step two is taken out from the thermal cycler and placed on an ice plate. A reaction system is formulated according to the following table:

| | |
|---|---|
| Reaction product from step two | 25 µL |
| NadPrep^{®} Universal Stubby Adapter | 2 µL |
| Ligation Buffer | 13 µL |
| DNA Ligase | 2 µL |
| Total | 42 µL |

### Step four: Purification of ligation product

The purification and recovery are performed using 0.5× Beads, and the purification process is as follows:
1. Firstly, 20 µL of NadPrep^{®} SP Beads are added to the ligation reaction product from step three, and the mixture is mixed well and incubated at 20-25°C for 10 min.
2. The PCR tube is centrifuged briefly and then placed on a magnetic rack for 5 min until the liquid becomes completely clear. The supernatant is aspirated using a pipette and discarded.
   Note: The liquid must be completely clear, and therefore the placement time needs to be adjusted for magnetic racks of different brands.
3. Then, 150 µL of 80% ethanol is slowly added along the side wall of the PCR tube, taking care not to disturb the magnetic beads. The PCR tube is let stand for 30 sec. The supernatant is aspirated using a pipette and discarded.
4. Step 3 is repeated once.
5. The PCR tube is centrifuged briefly and then placed on a magnetic rack. A small amount of residual ethanol is removed using a 10 µL pipette tip, taking care not to aspirate the magnetic beads.
6. The lid of the PCR tube is opened and the PCR tube is let stand at 20-25°C for about 2-3 min until the ethanol is completely evaporated.
7. The PCR tube is taken out and 44 µL of Nuclease Free Water is added to the PCR tube. The magnetic beads are suspended evenly using a pipette and incubated at 25°C for 2 min.
8. The PCR tube is centrifuged briefly and then placed on a magnetic rack for 2 min until the liquid becomes completely clear. The supernatant is carefully transferred into a new 0.2 mL PCR tube using a pipette.

### Step five: Enzymatic cleavage by MSRE

1. MeEnzyme & MeBuffer is taken out and placed on ice for natural dissolution (NEB restriction endonuclease), mixed well and centrifuged briefly for later use.
2. The reaction system is formulated in a 0.2 mL PCR tube placed on ice according to the following table, using MSRE. The system is as follows:

| | |
|---|---|
| MeEnzyme (MSRE) | 1 µL |
| MeBuffer | 5 µL |
| Purified ligation product from step four | 44 µL |
| Total | 50 µL |

3. The following reaction programs are initiated on a thermal cycler and the PCR tube is placed in the thermal cycler when the temperature is stabilized to 37°C: 37°C for 1 h; and 80°C for 20 min.

### Step Six: Purification of cleavage product

The purification and recovery are performed using 1× Beads. The purification process is found in step four, and 20 µL of NucleaseFree water is used for elution.

### Step seven: PCR amplification of cleavage product

1. 2× HiFi PCR Master Mix and NadPrep^{®} Universal UDI-Index Primer Mix are taken out and placed on ice for natural thawing, mixed well and centrifuged briefly.
2. PCR Amplification Mix is formulated in a 0.2 mL PCR tube placed on ice according to the following table:

| | |
|---|---|
| **Purified cleavage product from step six** | 20 µL |
| NadPrep^{®} Universal UDI-Index Primer Mix | 5 µL |
| 2× HiFi PCR Master Mix | 25 µL |
| Total | 50 µL |

3. The PCR tube is placed in a thermal cycler, and the following programs are initiated:

| | | |
|---|---|---|
| 98°C | 2 min | |
| 98°C | 15 s | |
| 60°C | 30 s | 6-8 cycles |
| 72°C | 30 s | |
| 72°C | 2 min | |
| 4°C | Hold | |

### Step eight: Purification of amplified product

The purification and recovery are performed using 1× Beads. The purification process is found in step four, and 20 µL of NucleaseFree water is used for elution.

### Step nine: Library hybridization

1. A hybridization reaction system is formulated according to the following table:

| | |
|---|---|
| Sample | 18 µL |
| µCaler NanoBlockers | 2 µL |
| µCaler ReMe Panel | 2 µL |
| µHyb #1 | 30 µL |
| µHyb #2 | 6 µL |

2. The hybridization reaction mixture is mixed well by vortexing for more than 10 sec, centrifuged briefly and then collected to the bottom of the PCR tube (avoid the generation of bubbles).
3. The PCR tube is placed in a thermal cycler and the following reaction programs are initiated: 98°C for 2 min; and 60°C for 1 h.

### Step ten: Library capture and elution

### Magnetic bead cleaning

1. Streptavidin Beads are vortexed for 15 sec to ensure complete well mixing.
2. n × 25 µL of Streptavidin Beads are taken and placed in a 0.2 mL centrifuge tube for mixing and washing (n represents the number of capture libraries and n < 5)
   Note: When n > 5, Streptavidin Beads should be distributed into multiple tubes for mixing and washing.
3. Streptavidin Beads are placed on a magnetic rack for standing for about 2 min until the liquid becomes completely clear. The supernatant is aspirated and discarded using a pipette.
4. The centrifuge tube is removed from the magnetic rack, and 100 µL of preheated Wash Buffer A is added. The mixture is mixed well by gently pipetting for above 10 times.
5. The centrifuge tube is placed on a magnetic rack for standing for about 2 min until the liquid becomes completely clear. The supernatant is aspirated and discarded using a pipette.
6. Steps 4 and 5 are repeated once.
7. The centrifuge tube is centrifuged briefly, and the entire Wash buffer A at the bottom of the tube is discarded using a 10 µL pipette tip. 8. Streptavidin Beads are suspended in n × 8 µL of uHyb #1. The mixture is mixed well by gently pipetting for above 10 times.

### Magnetic bead capture

1. After 1 hr of hybridization, the capture step is performed, and the thermal cycler is maintained in operation.
2. With the PCR tube remaining in the thermal cycler, 8 µL of the resuspended Streptavidin Beads are immediately added to each hybridization reaction solution. The mixture is mixed well by gently pipetting for above 10 times.
3. The mixture is incubated at 60°C for 10 min.
4. After the incubation, the PCR tube is removed from the thermal cycler and placed on a magnetic rack for standing for 2 min until the liquid becomes completely clear. The supernatant is discarded using a pipette (ensure that the supernatant is removed as thoroughly as possible).

### Elution

1. The PCR tube from the previous step is removed from the magnetic rack, and 150 µL of preheated Wash Buffer A is added. The mixture is mixed well by gently pipetting for above 10 times (avoid the generation of bubbles).
2. The PCR tube is placed on a magnetic rack for standing for 2 min until the liquid becomes completely clear. The supernatant is aspirated using a pipette and discarded. The PCR tube is removed from the magnetic rack, and 100 µL of preheated Wash Buffer A is added. The mixture is mixed well by gently pipetting for above 10 times. The reaction solution is transferred to a new PCR tube.
   Note: In this step, every effort should be made to avoid the generation of bubbles, as the contact between the bubbles and the tube cap may lead to a decrease in the on-target rate.
3. The PCR tube is placed in the thermal cycler and incubated at 60°C for 3 min.
4. The PCR tube is placed in the thermal cycler and incubated at 60°C for 3 min.
5. After the incubation, the PCR tube is removed from the thermal cycler and placed on a magnetic rack for standing for 2 min until the liquid becomes completely clear. The supernatant is discarded using a pipette.
6. 150 µL of Wash Buffer B placed at room temperature is added to the PCR tube. The mixture is mixed well by gently pipetting for above 10 times.
7. The PCR tube is placed on a magnetic rack for standing for 2 min until the liquid becomes completely clear. The supernatant is aspirated and discarded using a pipette.
8. The PCR tube is centrifuged briefly and then placed on a magnetic rack. A small amount of residual liquid is removed using a 10 µL pipette tip, taking care not to aspirate the magnetic beads.
9. Next, 22.5 µL of Nuclease Free Water is added. The mixture is mixed well by gently pipetting for above 10 times to resuspended the magnetic beads.

### Step eleven: PCR amplification of hybridization-captured library

1. Firstly, 2× HiFi PCR Master Mix and NadPrep^{®} Universal UDI-Index Primer Mix are taken out and placed on ice for natural thawing, mixed well and centrifuged briefly.
2. PCR Amplification Mix is formulated in a 0.2 mL PCR tube placed on ice according to the following table:

| | |
|---|---|
| Purified cleavage product from step six | 20 µL |
| NadPrep^{®} Primer Mix | 5 µL |
| 2× HiFi PCR Master Mix | 25 µL |
| Total | 50 µL |

3. The PCR tube is placed in a thermal cycler, and the following programs are initiated:

| | | |
|---|---|---|
| 98°C | 2 min | |
| 98°C | 15 s | |
| 60°C | 30 s | 10 cycles |
| 72°C | 30 s | |
| 72°C | 2 min | |
| 4°C | Hold | |

### Step twelve: Purification of amplified product

The purification and recovery are performed using 1× Beads. The purification process is found in step four, and 20 µL of NucleaseFree water is used for elution.

### Example 1: Test using fully methylated and completely unmethylated samples

Two standards, 100% Methylated DNA and 0% Methylated DNA (ZYMO), were used to construct different libraries. The library construction process was as follows. Briefly, 50 ng of each of the standards was taken for A-tailing end repair and adapter ligation. The adapter ligation products are divided into two groups. One group was cleaved with the MSRE Hha I and the other group was left uncleaved and served as a control group. PCR amplification was performed separately for the two groups of products, with the addition of Index sequences (FIG. 5A). The amplified products were subjected to hybridization capture using the µCaler hybrid Capture system. All the probes were designed to cover MSRE cleavage sites at a density of 10 KB per probe. The sequences of the capture probes used are shown in the table below. The detailed experimental process can be found in the experimental steps above.

| Probe number SEQ ID NO: | Sequence (5' biotin) |
|---|---|
| 1 | /biotin/CGTCGGTCGGGAGAGCGGTGGGAAGTATTCGCCTCCGTTCCTCACACCGACG |
| 2 | /biotin/CGTCGGTCGACAGGATGCGAGCGCCCTTTGGAGAAGTCGCGCCACCGACG |
| 3 | /biotin/CGTCGGTCGGCGGAAACGCTAACTCCCCGGCCAAGTGCAAATGCACCGACG |
| 4 | /biotin/CGTCGGTCGGCACACACGAGGGCCCGTCGCGCCCCCCGCCCTGCCACCGACG |
| 5 | /biotin/CGTCGGTCGGCCTCGCCCTCCACGTCCCTGCACCCCCAAGTCGCACCGACG |
| 6 | /biotin/CGTCGGTCGTAAGAACCCAGTCCCCGATCGGTTTCCTCTACGCCACCGACG |
| 7 | /biotin/CGTCGGTCGGGAGGGAGGGGGGGAAAGGGGGACGAGGGGCGGACACCGACG |
| 8 | /biotin/CGTCGGTCGGGGGGGTTTGGAAGCGCACTGCCACTTGCAAAGTCCACCGACG |
| 9 | /biotin/CGTCGGTCGACCCGAGCAATCACAGCCGGAGCGCGATTCGAATGCACCGACG |
| 10 | /biotin/CGTCGGTCGTGAACCGAAGACCTCGTAGGCGGGCCTCGGGGGGCACCGACG |
| 11 | /biotin/CGTCGGTCGGATGCCGTTGGCGGCGGCCAGCGCCAGCCCCTCGGCACCGACG |
| 12 | /biotin/CGTCGGTCGGTGCCGTAGAGCAGCTGCAGCTCGCGCGCCTCGTCACCGACG |
| 13 | /biotin/CGTCGGTCGGGCTCAGGTCGCCGCAGCCCGGGAGCCTCCCCGCCACCGACG |
| 14 | /biotin/CGTCGGTCGGCCCCAAGGCACGCGCGGCACAGCCATGAACACCCACCGACG |
| 15 | /biotin/CGTCGGTCGGATGCCAAGGAGTATCTGGCCCGGAGGGAAATCCCACCGACG |
| 16 | /biotin/CGTCGGTCGGAGTCCCGCGCGCTCTCCGTGCGCCCCGGCCGGCCACCGACG |
| 17 | /biotin/CGTCGGTCGGCGGCGGCTGCCGCGCACAGGCTTCCGACTCCAGCCACCGACG |
| 18 | /biotin/CGTCGGTCGCGGCCCGCCACTGAGCATGCCCAGCACGCCGGCCCACCGACG |
| 19 | /biotin/CGTCGGTCGGAGCGGAGGGGACAGCGGGGATCGTGAGCTCCGGCCACCGACG |
| 20 | /biotin/CGTCGGTCGGGGCGAGCGGGTGCGTCTGCCGCAGAGTCGGCACCCACCGACG |
| 21 | /biotin/CGTCGGTCGAAGGACATGGAGGTAAAGGACCCCGGAGGGAGACGCACCGACG |
| 22 | /biotin/CGTCGGTCGGCACGTGCGGCGCTCAGCTTAGGCTCTCGGAGGCACACCGACG |
| 23 | /biotin/CGTCGGTCGTGAGTTGGAAATCCCGACGGAAAGCACCCACAAGCCACCGACG |
| 24 | /biotin/CGTCGGTCGCACTCTGCGCTGGCCCACCCGCGTGCACGCCCACCACCGACG |
| 25 | /biotin/CGTCGGTCGGCGGAGCTAAGCAGGGGTGTTTGGAAGGCGCCCGCACCGACG |
| 26 | /biotin/CGTCGGTCGGCTGTTTGACTGCGTGGCTTCACTTGGCCCGCGCACCGACG |
| 27 | /biotin/CGTCGGTCGATCAGCCTCTCTGGCGGGGGATTCTCCCCGGACTCCACCGACG |
| 28 | /biotin/CGTCGGTCGGGCGGGCGGGGAGCCAGGCCCGAGCTGCGTTCTGCCACCGACG |
| 29 | /biotin/CGTCGGTCGAGCCATTGGTGGGCGCCGCGCTCTGCACTGAGCACACCGACG |
| 30 | /biotin/CGTCGGTCGTCGCGCCCCGCCGGCCCCTAGCCGCAGCCGCAGCCCACCGACG |
| 31 | /biotin/CGTCGGTCGGGCAGGCGGCAGGACGGGGATCCCAGGCTGCGGGGCACCGACG |
| 32 | /biotin/CGTCGGTCGACTCCGCGGCAGAGCCGCTGGGACCTGACCCGGGACACCGACG |
| 33 | /biotin/CGTCGGTCGGCCTTCGTCCCCGCGCGCACCTCCCCGGGTCGGGCCACCGACG |
| 34 | /biotin/CGTCGGTCGAAGGGGGGCTGGTGGAATCTGGCGGTCCCCAGCCACCGACG |
| 35 | /biotin/CGTCGGTCGCGTGTCCCGGGTCGGTGCGCTCGGCGCACCCGTGCACCGACG |
| 36 | /biotin/CGTCGGTCGGACAGTGCCCGGCGTCTGCTCCCACCCGCCCGCCCCACCGACG |
| 37 | /biotin/CGTCGGTCGGAACTTGCCGGTGGTGGGGTCGTAGTGGTTTCCGACACCGACG |
| 38 | /biotin/CGTCGGTCGTTGGTGACCACGTCGTCGAACTTGAGCACCTCGTACACCGACG |
| 39 | /biotin/CGTCGGTCGTTCATGCTGCCGCTTGAGGCCGGCGTAGAAGGCGCACCGACG |
| 40 | /biotin/CGTCGGTCGGGCGGCCCGGGCACCGCGAGCCGGCCGAGCTCCACACCGACG |
| 41 | /biotin/CGTCGGTCGGGAGCTACGTGACTACGTCCACCCGCACCTACAGCACCGACG |
| 42 | /biotin/CGTCGGTCGTGGGCAGCGCGCTGCGCCCCAGCACCAGCCGCAGCCACCGACG |
| 43 | /biotin/CGTCGGTCGGGCGCCGGGCCCGCGATCGGAGCCGGAGGCACCGCCACCGACG |
| 44 | /biotin/CGTCGGTCGAGCGGAAAGAGGGCTCCGCGGCGGCCAGGCTCAGCCACCGACG |
| 45 | /biotin/CGTCGGTCGTGCCGGGAAAATGCTGGATTTAACTCCCAGTCTGCCACCGACG |
| 46 | /biotin/CGTCGGTCGTCCCGGGGATGCCAGACTCCTGGGGACGCTGGGACACCGACG |
| 47 | /biotin/CGTCGGTCGGCGGCGCGGCGGGACACGCAGGACTCCCGCCTCCACCGACG |
| 48 | /biotin/CGTCGGTCGGCCCGGAATTCGTTGAGACGGAATCTCAGCGGATCCACCGACG |
| 49 | /biotin/CGTCGGTCGTGGGGAGAAGCGCTGCGGAAAGGGGCGACTCCGACACCGACG |
| 50 | /biotin/CGTCGGTCGAGATGGCCCTGTCCCGGCGCCCCAGGTCGTCGCGCCACCGACG |
| 51 | /biotin/CGTCGGTCGGCAGCTGCGGTAGTCACTGCGCCTCCCCGCCCCCACACCGACG |
| 52 | /biotin/CGTCGGTCGCTGGATGCCCCCCTTCCCTCTCCCGGCCAGACTCCACCGACG |
| 53 | /biotin/CGTCGGTCGCGGTGCTGTGTGCCTGGCCGGAGCGTTTTGAAACACCGACG |
| 54 | /biotin/CGTCGGTCGTCCGGGGCCGCTCGGCGGCGCTGCGATTGGCCGCGCACCGACG |
| 55 | /biotin/CGTCGGTCGGGGTAACCTCTATGCAAATGAGGCCGCGCCGCCCACCGACG |
| 56 | /biotin/CGTCGGTCGGCGGGCTCGGCCCGGTCCCTCCGCTGGGGGGTTACACCGACG |
| 57 | /biotin/CGTCGGTCGAGCCGGCCGGCAGCGAGATACCCAGAGGCCCGTTCACCGACG |
| 58 | /biotin/CGTCGGTCGCACCGCGTTGCCATAGCAACGGCCTGGCCGGCTGCACCGACG |
| 59 | /biotin/CGTCGGTCGGAGGTGCCGGCTGCACAACGGCGCCCACGTGACCCCACCGACG |
| 60 | /biotin/CGTCGGTCGGTGCAGCCCCAGGCGCGCCCAAGGGCAGCTCGCCCACCGACG |
| 61 | /biotin/CGTCGGTCGGAGAAGGACCTAAAACCCCGTCTGGGGCACAGAGGCACCGACG |
| 62 | /biotin/CGTCGGTCGACATCTGGGGAAACCGAGGCGCAGAGCGGGACGGCCACCGACG |
| 63 | /biotin/CGTCGGTCGTGGGCCTGGCGGGAAGTCGCGCAAGGAGGCCGGCACACCGACG |
| 64 | /biotin/CGTCGGTCGGGGCGCACCCGGCCCACCCTGTCCCTTCCAGGCCGCACCGACG |
| 65 | /biotin/CGTCGGTCGGCTGCAGCACAGGCTGCAGGGGGCGGGCAGCGCCGCACCGACG |
| 66 | /biotin/CGTCGGTCGTCTAGCGGGGCAGGCGAGCGGAGCAGCAGCGTTCCACCGACG |
| 67 | /biotin/CGTCGGTCGAGCCCCGGCCGCGGCCCCTGAGAGGCGCGCACTCGCACCGACG |
| 68 | /biotin/CGTCGGTCGGCCTCCCTGTGCCAGCCGGCTGTCGACCCAGGTTACACCGACG |
| 69 | /biotin/CGTCGGTCGAAGCCCGAGGTCGGGGCGTTACCCCAAGGGCCCTCCACCGACG |
| 70 | /biotin/CGTCGGTCGGCTTCCCCTCCGAGGGCAGAGAGGCGTCCGCGCCCCACCGACG |
| 71 | /biotin/CGTCGGTCGGGGGCTGCGGGGTCCGTGGAACCTTTCCGGGAGGCCACCGACG |
| 72 | /biotin/CGTCGGTCGGCCGCGGGCTGCTGGGCGCGGAGGCTGGAGCCGCGCACCGACG |
| 73 | /biotin/CGTCGGTCGAGGTTCGGCTGCTGAAGCGGCCACTGCCTCCCAGACACCGACG |
| 74 | /biotin/CGTCGGTCGGATCGATGGAGAGAAGGCGGGCAAGACGCCGGGAACACCGACG |
| 75 | /biotin/CGTCGGTCGGCATTCCTCCTCAACCGAGTGCCACAACCGCCCTCCACCGACG |
| 76 | /biotin/CGTCGGTCGGAAGTGCCCCGGGGCTTCGAGCATCACCTCGCGGCACCGACG |
| 77 | /biotin/CGTCGGTCGAGCTGAGACCGGCGGCCGACGGCCAGCCCTCAGGGCACCGACG |
| 78 | /biotin/CGTCGGTCGGGTCACAAGTCAGCGCCCAAGCAAGTCAAGCGACCACCGACG |
| 79 | /biotin/CGTCGGTCGGCTCGTCTTCGCCCGAACTGATGCGCTGCAAACGCACCGACG |
| 80 | /biotin/CGTCGGTCGGGCTCAACTTCAGCGGCTTTGGCTACAGCCTGCCGCACCGACG |
| 81 | /biotin/CGTCGGTCGGAGCGCAACCGCGTCAAGTTGGTCAACCTGGGCTCACCGACG |
| 82 | /biotin/CGTCGGTCGCACCCTTCGGGAGCACGTCCCCAACGGCGCGGCCCACCGACG |
| 83 | /biotin/CGTCGGTCGAAGAAGATGAGTAAGGTGGAGACACTGCGCTCGGCACCGACG |
| 84 | /biotin/CGTCGGTCGTGGCCCTGGTTGGGTCCGCCCGCAGCGAGGGCGCACCGACG |
| 85 | /biotin/CGTCGGTCGAGACCTGCGAAGAAGTTCGGAAACTTTTCCAGTGGCACCGACG |
| 86 | /biotin/CGTCGGTCGGCTGCTGGGAGCTGTCAGGGGGCTGCCGGATTCGCCACCGACG |
| 87 | /biotin/CGTCGGTCGGCCGAGGCTGGGAGCGCGGTGATGGCCGGTCCCCGCACCGACG |
| 88 | /biotin/CGTCGGTCGAGCAGAGGGCAGCGGCGGCGGGACCGGGGACTCTGCACCGACG |
| 89 | /biotin/CGTCGGTCGCAATGGGTAAAGCCAGGAGGAAGCCCGTGCCTGGCACCGACG |
| 90 | /biotin/CGTCGGTCGAGGCGGGCAGGAGGGGAGGAGTAGGGAGGCGAGAGCACCGACG |
| 91 | /biotin/CGTCGGTCGGCGGGCGGGAGGCGCCACGGCCTCTCAGACGCTGGCACCGACG |
| 92 | /biotin/CGTCGGTCGCCGCAGCCGAGAAGCCAGAGAGAAAGTTCCCGGGCACCGACG |
| 93 | /biotin/CGTCGGTCGGCAGTAAGCGAGTAAGCGAGCCCTGGAGACCGGGCACCGACG |
| 94 | /biotin/CGTCGGTCGCTGGGTGTGCGGTCGGCTCAGTGCCACGCGGTGACACCGACG |
| 95 | /biotin/CGTCGGTCGTAGGCATGGTTATCAACCCACGCCAAGGCGGTTGCACCGACG |
| 96 | /biotin/CGTCGGTCGGCTGCCTCGCCCCGGAGCCCGGAGGAGGGGAGCCGCACCGACG |
| 97 | /biotin/CGTCGGTCGACCCGGGGAAGAAGCGGAGGACGCCGATCTGGCCCACCGACG |
| 98 | /biotin/CGTCGGTCGTGCGTTGCGCGCTCCAACCCTCTGCTTGGCCGCCCACCGACG |
| 99 | /biotin/CGTCGGTCGACAAATCCCCGAGGCGGGGAGACTTTCAGGGCATCCACCGACG |
| 100 | /biotin/CGTCGGTCGGATGCTGAAGCCTCGCGGTCCCCATTCCCAAGCCCCACCGACG |
| 101 | /biotin/CGTCGGTCGCCGTGCGCCGCCTGCGCGTGGCGCAGTTAATTTGCACCGACG |
| 102 | /biotin/CGTCGGTCGGTCTTGAGGTGGGGAGGGGAGAAATGGGAAGAGGCACCGACG |
| 103 | /biotin/CGTCGGTCGAGTAGCGGTTTTAGCCCGCTCTGCGGCTGCGAGGCACCGACG |
| 104 | /biotin/CGTCGGTCGAGATCCGAGATTAACCTCTCCCGCGATAGGTGAAGCACCGACG |
| 105 | /biotin/CGTCGGTCGTTGGGGCGACGGGGCCAGGCGCGGGGGACGGCTGCACCGACG |
| 106 | /biotin/CGTCGGTCGGGCTGGGAGGGCGCGCGGAGGAGACACCGCTGAGGCACCGACG |
| 107 | /biotin/CGTCGGTCGACGCCGCTGAGGGCGCCACGCGGGGGGCGCGGCTGCACCGACG |
| 108 | /biotin/CGTCGGTCGAGTGCGCACTTGAGGCGCGGGACGCACCGGCTGTGCACCGACG |
| 109 | /biotin/CGTCGGTCGTCCCAGGGGCTGAGACTCGCGCCCCGCCCCGCAGCCACCGACG |
| 110 | /biotin/CGTCGGTCGTCGGCTCGCCGGGCCGGGCCCCTCTCCGGAACCTCCACCGACG |
| 111 | /biotin/CGTCGGTCGGCGGCTGGAGGACGCGCTGCTGCGGATGCGCGAGGCACCGACG |
| 112 | /biotin/CGTCGGTCGTACGGGATACAGGCCGAGGAGCGGCAGGTCCGTGCCACCGACG |
| 113 | /biotin/CGTCGGTCGGGGGATGGCGCGCTGACCCCATACCCGCTGCCGTCCACCGACG |
| 114 | /biotin/CGTCGGTCGCGGCTGCGGGGCCGCCGCCGAGCGAGGGCGAGGACACCGACG |
| 115 | /biotin/CGTCGGTCGGCACCGTGCGCTTCGCCCGCAAAGGCGCCCTCCGGCACCGACG |
| 116 | /biotin/CGTCGGTCGGAAGAACGTGCATGAGGTCAAGAACCACAAATTCACACCGACG |
| 117 | /biotin/CGTCGGTCGGATGGCCGCGCCGCCCCGGGGGAGCCGGAGCCGACACCGACG |
| 118 | /biotin/CGTCGGTCGTGGCCGCCGCCCGCGCGCACTCACCAAGCCCGCTGCACCGACG |
| 119 | /biotin/CGTCGGTCGTCCTCCTCGCACGAGTACCAGATGCCGGTGTGGACACCGACG |
| 120 | /biotin/CGTCGGTCGTCCCTGGGCCGGGACTGGAGCGCAAACGGTTGGGACACCGACG |
| 121 | /biotin/CGTCGGTCGTGTCGGTGTCTGCCCGCCCGTCTGTGCGTCTGTCCACCGACG |
| 122 | /biotin/CGTCGGTCGTGTCGGTGTGAGCGTCTGAGGCGATGGGGAAGACACACCGACG |
| 123 | /biotin/CGTCGGTCGGGGCGCTTCTAGTCGGACAAAATGCAGCCGAGAACACCGACG |
| 124 | /biotin/CGTCGGTCGCGCTCGTTCTGTGCGTTCTCCTGTCCCAGGTAGGCACCGACG |
| 125 | /biotin/CGTCGGTCGAGAGGGGCTGCCGGGCGCGCTCTGCGCCCCGTTTCCACCGACG |
| 126 | /biotin/CGTCGGTCGTGGGAGCAGGGTGTCCGAAGCGCGGGTACCGATCCACCGACG |
| 127 | /biotin/CGTCGGTCGGGCAGCGCGGGGCACGCGGCGCGCGGGCGAGTCTGCACCGACG |
| 128 | /biotin/CGTCGGTCGTGCGCGCGCGGACGCCTTGGTTCCCGGAGCCCAGCACCGACG |
| 129 | /biotin/CGTCGGTCGCCACCTTCTCAGTCCGGGACGCGAGACGGGAACGCACCGACG |
| 130 | /biotin/CGTCGGTCGGCCCTGGCGCCCCAACGCTGCACCACCGTGGGTCCACCGACG |
| 131 | /biotin/CGTCGGTCGGAGCCGTGCTCGCAGTCGCTGAGATCCTCGGTGCGCACCGACG |
| 132 | /biotin/CGTCGGTCGGCGTCTCTCGCCGTCCCCTGGGCGCGGGCCAGGCGCACCGACG |
| 133 | /biotin/CGTCGGTCGGAGGAGGGGGGCGCTCCGGTCGTGTGCCCAGGACCACCGACG |
| 134 | /biotin/CGTCGGTCGCCCCAGCGGCCACTCGGGCCCCAGCCCCCCAGGCCACCGACG |
| 135 | /biotin/CGTCGGTCGTGGAGATTTAGAGCCCGGGAATGAGGCGCCGTGTCCACCGACG |
| 136 | /biotin/CGTCGGTCGTAGGAATCTCGCGCCCGGGGAGCGCTGAGGGACCGCACCGACG |
| 137 | /biotin/CGTCGGTCGGAGCCGGGTGGGGAGCGGGCCTCGGGGCGCCCGGCCACCGACG |
| 138 | /biotin/CGTCGGTCGGCAGGGCCGGAAGGAGGCCCCTGCCCCGTCCAAGGCACCGACG |
| 139 | /biotin/CGTCGGTCGGCACCAGCTGCCCCGCCGCCCGCCCGGACCCAGCCCACCGACG |
| 140 | /biotin/CGTCGGTCGGCCTCATTGCTGACGAGACCCCGCCCTGCTACTCCCACCGACG |
| 141 | /biotin/CGTCGGTCGGGCCGGAGAAGGCGCCGCTGGGGCCGCCCGGAGGACACCGACG |
| 142 | /biotin/CGTCGGTCGCCTCTGCACGGGCCCGTGGAGACGCTTCCTGCGCACCGACG |
| 143 | /biotin/CGTCGGTCGAACCTTCTGGAATCCACCGCCCCCCTTTCCGCCCGCACCGACG |
| 144 | /biotin/CGTCGGTCGCGCGCCCGCCCCCAGGAGGGCCTCCGCGAGCCGGCACCGACG |
| 145 | /biotin/CGTCGGTCGGCACACCCCGAGGCGGTCCCGGCTGCACAACTTGGCACCGACG |
| 146 | /biotin/CGTCGGTCGGAGTTGCTCCGTTTCCTCATTTTGGGGGCGAAGACACCGACG |
| 147 | /biotin/CGTCGGTCGGCCGGCGGCGGCCAGTAGCGGCGCCGTGTGCAGGGCACCGACG |
| 148 | /biotin/CGTCGGTCGAGCGCCGCCTGCGCCGCGTTCAGCACGAAGAGCTCCACCGACG |
| 149 | /biotin/CGTCGGTCGTCCAGCTCAGGCGCAGCAGCGCCACCTGGTCGGCCCACCGACG |
| 150 | /biotin/CGTCGGTCGGGCGCCCGCATTGGTGCTGACATAATTTCCTGACCCACCGACG |
| 151 | /biotin/CGTCGGTCGTGACCCGTATTGTCTCGCGATTAAAGGTAAAAAACGCACCGACG |
| 152 | /biotin/CGTCGGTCGGCTTTTTCATCCCACTGGGGTAAAACGCCCTTTTACACCGACG |
| 153 | /biotin/CGTCGGTCGAGTGGGGCCGCGGGGCCTGCTGGGAGGTGTTGTCCCACCGACG |
| 154 | /biotin/CGTCGGTCGGGAAACGTCGCTGGCGCGGAGGGATGGTTCGGCGCCACCGACG |
| 155 | /biotin/CGTCGGTCGTAGGCGTCTGTCACAGACCTATCTGCGGGTCGCCCACCGACG |

The library construction output is as shown in FIGS. 5B and 5C. As can be seen from the library construction output, for the library construction of the endonuclease cleavage group of the 0% methylation standard, both the library construction yield and the cleavage yield were the lowest. Targets containing restriction sites were not methylated and could be cleaved by MSREs. Therefore, the library construction yield was lower than that of the non-cleavage experimental group and the experimental group with a methylation level of 100%. The on-target rate of the endonuclease cleavage group of the 0% methylated standard was approximately 10%, whereas that of other experimental groups was approximately 50%; for the endonuclease cleavage group of the 0% methylated standard, 0.2× Mean was approximately 35% and 0.5× Mean was approximately 25%. In contrast, for the other experimental groups, 0.2× Mean was approximately 95% and 0.5× Mean was approximately 80%; the average depth (rmdup) of the endonuclease cleavage group of the 0% methylated standard was approximately 160, whereas that of the other experimental groups was approximately 1200 (FIG. 6). The basic quality control after sequencing indicated that MSREs cleaved samples with methylation levels of 0, with unmethylated sites cleaved; the MSREs cleaved samples with methylation levels of 100%, with methylation-modified sites not cleaved.

FIG. 7 shows sequencing depth and corresponding methylation levels at CpG sites related to MSRE in target regions of different libraries. Panel A shows the sequencing depths at the same CpG sites in cleavage and non-cleavage libraries from 100% methylated sample, ranging from hundreds to thousands of reads. Panel B shows the sequencing depths at corresponding CpG sites in cleavage and non-cleavage libraries of the sample with a 0% methylation level, ranging from hundreds to thousands of reads, which is consistent with the results of the experimental group of the sample with a 100% methylation level. The sequencing depths at corresponding CpG sites in the cleavage library of the sample with a 0% methylation level were all basically 0. The MSREs cleaved unmethylated sites, with the vast majority of sites cleaved and methylated sites not cleaved.

FIG. 7C shows the results of methylation levels at CpG sites related to MSRE in different samples. The calculation was carried out using the formula: cleavage depth/control depth. The methylation level detected in the 0% Methyl sample was close to 0 and the methylation level detected in the 100% Methyl sample was close to 100%. The detection results were in line with the expectations.

### Example 2: Test of other MSREs using fully methylated and completely unmethylated samples

Different libraries were constructed using two standards, 100% Methylated DNA and 0% Methylated DNA (ZYMO), with the library construction process being the same as that in Example 1. In this example, the MSRE Aci I was selected and tested for cleavage efficiency on standards with different methylation levels. The sequencing depth and corresponding methylation levels at CpG sites related to the MSRE Aci I in the target region of the library are calculated, with results as shown in FIG. 8. The methylation level detected in the 0% Methyl sample was close to 0 and the methylation level detected in the 100% Methyl sample was close to 100%. The detection results were in line with expectations. This hybridization capture system is suitable for different types of MSREs. The selection of the endonuclease is mainly related to the types of restriction endonucleases contained in the target gene.

### Example 3: Test using samples with different methylation levels

100% Methylated DNA and 0% Methylated DNA (ZYMO) were mixed at a certain ratio to prepare methylation standards with methylation levels of 0%, 10%, 50% and 100%, respectively. Next, 50 ng of each of the standards was taken for A-tailing end repair and adapter ligation. The adapter ligation products were divided into two groups. One group was cleaved with the MSRE Hha I and the other group was left uncleaved and served as a control group. The groups are shown in the table below. PCR amplification was performed separately for the two groups of products, with the addition of Index sequences.

| Library name | lib1 | lib2 | lib3 | lib4 | lib5 | lib6 | lib7 | lib8 |
|---|---|---|---|---|---|---|---|---|
| Sample type | 0% Me | 0% Me | 10% Me | 10% Me | 50% Me | 50% Me | 100% Me | 100% Me |
| *Hha I* cleavage | + | - | + | - | + | - | + | - |

Amplified products were subjected to hybridization capture using the µCaler hybrid Capture system (with capture probes the same as those in Example 1). All the probes are designed to cover MSRE cleavage sites at a density of 10 KB per probe. Meanwhile, normalization was performed using the reference gene (by designing probes in regions that did not contain cleavage sites). The sequences of the normalized probes are shown in the table below.

| Probe number SEQ ID NO: | Sequence (5' biotin) |
|---|---|
| 156 | /biotin/CGTCGGTCGCCAGCGCCATAGCCCTTAGGACTATCGGTCACACACCGACG |
| 157 | /biotin/CGTCGGTCCTCGCGCTCCTGCTCCGGCTCCTCCATCTTGGCCCACCGACG |
| 158 | /biotin/CGTCGGTCGGAGCAGAGCGAGGTGTGTGCCTCCTTACCGCCTCACCGACG |
| 159 | /biotin/CGTCGGTCGTGCACTGGACGTCCTTCCCCAGCAGCCAGTTGAGCACCGACG |
| 160 | /biotin/CGTCGGTCAGACGGAGGCTGGTGGTGCAGCAGGCAGGCAAGACCACCGACG |
| 161 | /biotin/CGTCGGTCGATGCCGGGGACTACAGCTGCGAGGCCAGGGGCCACACCGACG |
| 162 | /biotin/CGTCGGTCGCACAGCGTTGTGCATCCAGGTCTAGCGTGTCTTCCACCGACG |
| 163 | /biotin/CGTCGGTCCTGTCTTCTGTGGATGAGGTATATGAATTTATCCCAAAGCACCGACG |
| 164 | /biotin/CGTCGGTCCGAGACCTCCCAGAGCCCGTGGTTCCCTGGAGCCACACCGACG |
| 165 | /biotin/CGTCGGTCTACGAAGGGTTCCTGCTCTGTGGGCAGCTCACGAACACCGACG |
| 166 | /biotin/CGTCGGTCCAATAATGCACATGGAGAAAGTTCACCTACAATAAAGAGGCACCGACG |
| 167 | /biotin/CGTCGGTCTGTATTGGTAACATTTTATATCGAATTCCTGTTCTATGTCACCGACG |
| 168 | /biotin/CGTCGGTCAGATGTCCCCTCCCTGTCCGTCCCCGCACCTGGAGCACCGACG |
| 169 | /biotin/CGTCGGTCTGGTGGTGCATGCCCGAACCCAGCACTTCCTTGAACACCGACG |
| 170 | /biotin/CGTCGGTCCAACTGGCCAACCTAGAGCCCCCCTGGTAAAAGCCACCGACG |
| 171 | /biotin/CGTCGGTCTCTTGGTGGAATAGATGTTAATTAGTTTTTTTATTACTCCACCGACG |
| 172 | /biotin/CGTCGGTCGGAAACAATTCTGTGTTCACCCTCACCCTGCAGGCCACCGACG |
| 173 | /biotin/CGTCGGTCGGCCTCTCAGCCATCAAGACACCGTATCCTACCTCCACCGACG |
| 174 | /biotin/CGTCGGTCACGTGCTGAGCGCACGCACGTGGCGCCTGCTCACCCACCGACG |
| 175 | /biotin/CGTCGGTCGATGCTGCTCCAGCGCCCGCGCGAGTTGCGGATCGCACCGACG |
| 176 | /biotin/CGTCGGTCGTAGTTGACAAATACATTTAGTGATGTCTTACTTTTTTGGCACCGACG |
| 177 | /biotin/CGTCGGTCATCTCCTACCAGTGTATCCTTCACGACAGACGCACCACCGACG |
| 178 | /biotin/CGTCGGTCCGAGCGATGATGACACCAAATCCATGTGTCCACCCCACCGACG |
| 179 | /biotin/CGTCGGTCGGGACCCAGGAGGGCACAGCCAAGGAATGAGCCCCACCGACG |
| 180 | /biotin/CGTCGGTCCTCCTGGTCAGCAGCCTCTTAGAGAACCTGCTGGACACCGACG |
| 181 | /biotin/CGTCGGTCTATAGAACCATCATCATGCAAGATGAGAGCAAGGAGCACCGACG |
| 182 | /biotin/CGTCGGTCGAACAGTAAACAGTGGTTCTGACTGGTGAAATGATAGTTACACCGACG |
| 183 | /biotin/CGTCGGTCGTTGAAAGGACCAGCACTTTATTTATTGCTTTAGCCATTCACCGACG |
| 184 | /biotin/CGTCGGTCAGCCTGCACGGGAGTCAGAGGCACTCAGCAATGCCCACCGACG |
| 185 | /biotin/CGTCGGTCTGGCTCTGTTACGAACGGCTGAAATCAAAACCCCCACCGACG |
| 186 | /biotin/CGTCGGTCTCACCGTGTTCTCCAGACCATGCATGTTCCTCCGCCACCGACG |
| 187 | /biotin/CGTCGGTCACCGCCCCTTCAGGGATGGGGCTGTAGGAGTCAAGCACCGACG |
| 188 | /biotin/CGTCGGTCTCTCGGTGTCTCCCCAGGTGCAAGTGCAACCTGCACACCGACG |
| 189 | /biotin/CGTCGGTCGCCAACCTGTGCTCCATGCGCGAGGGCAGCCTGCACACCGACG |
| 190 | /biotin/CGTCGGTCAAGCCCACTCTTCTACTCCCCGAACGTGCCTTCCCCACCGACG |
| 191 | /biotin/CGTCGGTCCTCTGACGGCGACAGTAGCTCCGTGGACAGCGATGCACCGACG |
| 192 | /biotin/CGTCGGTCCTGGAGGAGCACGGAAAAGACCTGGAAATCATGCACACCGACG |
| 193 | /biotin/CGTCGGTCATCCTCACCAGGGTGAATGACAGAGTTGCCAGGCACACCGACG |
| 194 | /biotin/CGTCGGTCGCATAGGAGTAGGCCAGCCAGCGGAACACGTGGTCCACCGACG |
| 195 | /biotin/CGTCGGTCTCGGGGGTGGGGGTGTGTTCCTGCGTCTTCCAGGGCACCGACG |
| 196 | /biotin/CGTCGGTCCGGGAATGCCGAGTAAGTGTTAATTTTATGGTCCCCACCGACG |
| 197 | /biotin/CGTCGGTCATCACTTCTGGAAAATGAATAGATAGATGTGTGGGCTTTACACCGACG |
| 198 | /biotin/CGTCGGTCGTGCCTCAATGATCTTTTTTCCCCACCTTCATTTTTCTCACCGACG |
| 199 | /biotin/CGTCGGTCCTTCAGCGGCATCTGCTAGCTCGTCTACCCCTGTCACCGACG |
| 200 | /biotin/CGTCGGTCCATGTTTTAGGGAACTCTGCCCTATAAACACTCATAGACACCGACG |
| 201 | /biotin/CGTCGGTCGGTTTCTCTCTATGTTGCAGTCCCTCTGTCGTGAACACCGACG |
| 202 | /biotin/CGTCGGTCCGGCATTCCTGTGGTAGTGGCCTGAGAACACGACCACCGACG |
| 203 | /biotin/CGTCGGTCTGACACCTGCAGAGAAGGGAAAAAGTCATTAGGGGCACCGACG |
| 204 | /biotin/CGTCGGTCACATGTCCCTGCGTCACAGGCACCTGCAGAGCCCCCACCGACG |
| 205 | /biotin/CGTCGGTCTGTGTAAGGCGTTCCGGCACGTCAAGGTGGACACACACCGACG |
| 206 | /biotin/CGTCGGTCTGAAGATACAAACAACCACAGCAGCCTGCACTTACCACCGACG |
| 207 | /biotin/CGTCGGTCGTGTGGTCATCTTCTTCTTCCCCTTCTAGCACGACCACCGACG |
| 208 | /biotin/CGTCGGTCACGTTGCATCGACCCTCTCCACTCCGCGGAAGTAGCACCGACG |
| 209 | /biotin/CGTCGGTCCGACACCGTCTGATGTGTTGCGCAGGTGGTGGCACACCGACG |
| 210 | /biotin/CGTCGGTCCATTGGGATGGAGGGTGGGGAAGGAGTCACCCTGGCACCGACG |
| 211 | /biotin/CGTCGGTCCCTCCTCCGCCTGACTTACTTGATCCTAAAGTCACACCGACG |
| 212 | /biotin/CGTCGGTCCTCCCAGGGCTCCTTGGCCTCCTCCGCGGTGACCCACCGACG |
| 213 | /biotin/CGTCGGTCGGGACACAGGGCCGCATGAGCCTGGGCGGGGTCAGCACCGACG |
| 214 | /biotin/CGTCGGTCCGCCCTCCGAGGAGGGCCCGGGCGGGGTGGGCGCCCACCGACG |
| 215 | /biotin/CGTCGGTCCGTCCTCAGGCTCCAGGCTGGGAGGAGAGAGCGTCACCGACG |
| 216 | /biotin/CGTCGGTCGCTGGGAGTGGAGCTGGGGGTCAGAAGAGCAAGCACACCGACG |
| 217 | /biotin/CGTCGGTCAACGAAATTGATCAAACTTAGACTGCTTCACCTGTCTCCACCGACG |
| 218 | /biotin/CGTCGGTCGCGTGGACAGGCAGCGCCGCAGTGACGTCTCCATGCACCGACG |
| 219 | /biotin/CGTCGGTCTGGAGTCCTCCAGCGGCTTCAGGCGGTTCAGCTTCCACCGACG |

FIG. 9 shows the results of methylation levels of CpG sites related to MSRE in different samples. Calculation method: cleavage depth/control depth. The methylation level detected in the 0% Methyl sample was close to 0 and the methylation level detected in the 100% Methyl sample was close to 100%. The methylation level detected in the 10% Methyl sample was close to 10% and the methylation level detected in the 50% Methyl sample was close to 50%. The methylation level detected in the 100% Methyl sample was close to 100%. The detection results were in line with expectations.

Methylation levels of different samples were calculated for the cleaved samples, using non-cleaved samples with different methylation levels as controls, and the results are shown in Table 10. Calculation method for methylation level: For each site, normalization was performed using the reference gene (by designing probes in regions that did not contain cleavage sites) and the methylation level was calculated according to formula: normalized depth of each CpG site in cleavage sample/normalized depth of each CpG site in control sample. The methylation level detected in the 0% Methyl sample was close to 0% and the methylation level detected in the 10% Methyl sample was close to 10%. The methylation level detected in the 50% Methyl sample was close to 50%. The methylation level detected in the 100% Methyl sample was close to 100%. The test results showed that by adding the reference gene for normalization and using non-cleaved samples with different methylation levels as controls, the methylation levels at individual CpG sites, as calculated for the samples in the cleavage group, were all in line with expectations.

## Claims

1. A method for detecting a methylation status of a DNA molecule in a sample, comprising:
1) dividing the sample into two parts: a first sample and a second sample, wherein the first sample is treated with one or more methylation-sensitive restriction endonucleases (MSREs), such that an enzymatic cleavage reaction is performed in the absence of methylation of the DNA molecule at recognition sites of the MSREs, so as to obtain a cleaved sample; or the sample is treated with one or more methylation-dependent restriction endonucleases (MDREs), such that an enzymatic cleavage reaction is performed in the presence of methylation of the DNA molecule at recognition sites of the MDREs, so as to obtain a cleaved sample; the second sample is not treated with restriction endonucleases;
2) contacting one or more capture probes with the cleaved sample, wherein the capture probes target a target sequence comprising the recognition sites in the DNA molecule, so as to isolate the non-cleaved DNA molecule comprising the recognition sites from the cleaved sample; contacting the capture probes with the second sample, so as to isolate the DNA molecule comprising the recognition sites from the second sample; and
3) making a comparison with an amount of the DNA molecule comprising the recognition sites in the second sample, and determining an overall methylation status of DNA molecules in the sample according to an amount of the non-cleaved DNA molecule comprising the recognition sites; or sequencing the non-cleaved DNA molecule comprising the recognition sites and the DNA molecule comprising the recognition sites in the second sample, and determining methylation statuses of different DNA molecules in the sample according to a ratio of sequencing depths.

2. The method according to claim 1, wherein the capture probes comprise a target-specific sequence, a first probe-binding sequence at the 5' end of the target-specific sequence and a second probe-binding sequence at the 3' end of the target-specific sequence, the first probe-binding sequence being at least partially complementary to the second probe-binding sequence, such that when two or more capture probes bind to the target sequence in an adjacent manner, complementary binding between the adjacent capture probes is formed by the first probe-binding sequence and the second probe sequence.

3. The method according to claim 1 or 2, wherein the first probe-binding sequence and the second probe-binding sequence are 8-30nt in length.

4. The method according to any one of claims 1-3, wherein the target-specific sequence is 20-80nt in length.

5. The method according to any one of claims 1-4, wherein before step 1), the method further comprises constructing a sequencing library with the DNA molecule in the sample and, optionally, fragmenting the DNA molecule before constructing the sequencing library.

6. The method according to any one of claims 1-5, wherein the MSREs are selected from the group consiting of Hpa I, Hpa II, Hha I, Aci I, and any combination thereof.

7. The method according to any one of claims 1-6, wherein the MDREs are selected from the group consiting of FspE I, LpnP I, MspJ I, and any combination thereof.

8. The method according to any one of claims 1-7, wherein the sample comprises circulating tumor DNA (ctDNA) and/or genomic DNA (gDNA), or the sample is a formalin-fixed paraffin-embeded (FFPE) sample.

9. A kit for detecting a methylation status of a DNA molecule in a sample, comprising:
1) one or more MSREs that cleave the DNA molecule in the absence of methylation of the DNA molecule at the recognition sites of the MSREs; or one or more MDREs that cleave the DNA molecule in the presence of methylation of the DNA molecule at the recognition sites of the MDREs; and
2) one or more capture probes that comprise a target-specific sequence, a first probe-binding sequence at the 5' end of the target-specific sequence and a second probe-binding sequence at the 3' end of the target-specific sequence, the first probe-binding sequence being at least partially complementary to the second probe-binding sequence, such that when two or more capture probes bind to a target sequence comprising the recognition sites in the DNA molecule in an adjacent manner, complementary binding between the adjacent capture probes is formed by the first probe-binding sequence and the second probe sequence.

10. The kit according to claim 9, further comprising an enzyme and an adapter molecule for constructing a sequencing library for the DNA molecule.

11. The kit according to claim 9 or 10, wherein the first probe-binding sequence and the second probe-binding sequence are 8-30nt in length.

12. The kit according to any one of claims 9-11, wherein the target-specific sequence is 20-80nt in length.

13. The method according to any one of claims 9-12, wherein the MSREs are selected from the group consisting of Hpa I, Hpa II, Hha I, Aci I, and any combination thereof.

14. The method according to any one of claims 9-13, wherein the MDREs are selected from the group consisting of FspE I, LpnP I, MspJ I, and any combination thereof.

15. The method according to any one of claims 9-14, wherein the sample comprises ctDNA.
